# EUROPEAN PATENT APPLICATION

(11) **EP 4 191 493 A1**
(43) Date of publication of application: **07.06.2023**
(21) Application number: 22159486.4
(22) Date of filing: 01.03.2022
(51) Int. Cl.: G06Q 10/10, G06Q 10/06, G16H 40/20, G16H 80/00

(54) **SCHEDULING SYSTEM AND METHOD INCORPORATING UNSCHEDULED INTERRUPTIONS**

(30) Priority: 03.12.2021 US 202163285509 P
(71) Applicant: Koninklijke Philips N.V., 5656 AG Eindhoven (NL)
(72) Inventor: AMTHOR, Thomas Erik, Eindhoven (NL); NORDHOFF, Tanja, Eindhoven (NL); SCHMIDT, Joachim Dieter, Eindhoven (NL)
(74) Representative: Philips Intellectual Property & Standards

(57) **Abstract**

A non-transitory computer readable medium (26s) stores instructions readable and executable by at least one electronic processor (14s) to perform a method (100) managing a schedule (42) of a medical professional. The method includes maintaining a schedule of tasks for the medical professional, the schedule including time intervals (44) with planned tasks; maintaining or receiving other schedules of tasks for others that interact with the medical professional; computing a likelihood of interruption of the medical professional as a function of time from the maintained or received other schedules; and outputting, on a display device (24, 36) of an electronic processing device (12, 8) operable by the medical professional, a representation (40) of the schedule of the medical professional with an indication of the likelihood of interruption as a function of time in the schedule.

## Description

### FIELD OF THE INVENTION

The following relates generally to the scheduling arts, remote imaging assistance arts, remote imaging examination monitoring arts, radiology scheduling arts, and related arts.

### BACKGROUND OF THE INVENTION

Radiologists are often interrupted in their work by ad-hoc (i.e., unplanned, and hence unscheduled) interruptions, such as requests and communication from a local technologist. For example, a radiologist may be asked to do an image quality check or answer a question during a complicating imaging examination. In some setups, a remote lead technologist will experience similar disruptions by unscheduled requests. Both the radiologist and the remote lead technologist generally have a schedule of fixed or known tasks and will have some freedom to decide how to distribute other activities, such as concentrated reading time or breaks.

However, radiologists or remote technologists do not know when to expect unscheduled tasks, which can interrupt scheduled activities. This makes it difficult for them to plan and manage their day. Interruptions can be a major source of stress for the staff members.

In addition, local technologists do not know when remote technologists or radiologists will most likely be available or occupied during the day. This makes it difficult for them to decide when to approach a remote technologist or radiologist without disrupting their work.

More generally, medical professionals and other workers often maintain a schedule on an electronic calendaring system implemented as a mobile device calendaring application (e.g., cell phone app), computer-based calendaring program, cloud-based calendaring service, or the like. Some commercially available electronic calendaring products include, by way of non-limiting illustrative example, Google Calendar (available from Google LLC), Microsoft Outlook (available from Microsoft Corp.), and Apple Calendar (available from Apple Corp.). In the medical domain, calendaring systems are sometimes integrated into medical workflow platforms that manage overall department- or hospital-wide operations. Such calendaring systems enable the user to maintain a personal calendar into which the user can input, revise, and delete events with designated time blocks. Some such calendaring systems include integration across users so that one user can create an event (i.e., task) that populates to other users' calendars. For example, a radiology department manager may create a calendar event that is distributed to the calendars of all radiologists in the department. Some calendaring systems integrate even more widely, so that for example a hospital worker can send out a calendar event for a medical appointment to the calendar of a patient, even if the patient's calendar is a different product than that used by the hospital.

In spite of these advanced capabilities and integrative aspects, some deficiencies remain. Notably, electronic calendaring systems are typically unable to accommodate unscheduled interruptions. This can be problematic as an unplanned (and hence unscheduled) interruption can disrupt the smooth flow of events scheduled by the calendar

The following discloses certain improvements to overcome these problems and others.

### SUMMARY OF THE INVENTION

In one aspect, a non-transitory computer readable medium stores instructions readable and executable by at least one electronic processor to perform a method managing a schedule of a medical professional. The method includes maintaining a schedule of tasks for the medical professional, the schedule including time intervals with planned tasks; maintaining or receiving other schedules of tasks for others that interact with the medical professional; computing a likelihood of interruption of the medical professional as a function of time from the maintained or received other schedules; and outputting, on a display device of an electronic processing device operable by the medical professional, a representation of the schedule of the medical professional with an indication of the likelihood of interruption as a function of time in the schedule.

In another aspect, a non-transitory computer readable medium stores instructions readable and executable by at least one electronic processor to perform an electronic calendar method including estimating likelihoods of interruption of an owner of an electronic calendar for time blocks of a calendar based on historical information on interruptions of the owner of the electronic calendar, the electronic calendar including tasks assigned for the owner of the electric calendar to time intervals of the electronic calendar; and displaying, on a display device controlled by the at least one electronic processor, a representation of the electronic calendar including the tasks assigned to the owner of the electronic calendar annotated to the corresponding time intervals and further including indications of the computed likelihoods of interruption annotated to the corresponding time blocks.

In another aspect, an electronic calendar method includes maintaining an electronic calendar including assigning tasks for an owner of the electric calendar to time intervals of the electronic calendar; estimating likelihoods of interruption of the owner of the electronic calendar for time blocks of the calendar based on schedules of tasks for others that interact with the owner of the electronic calendar; and displaying, on a display device controlled by the at least one electronic processor, a representation of the electronic calendar including the tasks assigned to the owner of the electronic calendar annotated to the corresponding time intervals and further including indications of the computed likelihoods of interruption annotated to the corresponding time blocks.

One advantage resides in estimating ad-hoc task requests for remote experts to assist a local operator performing a medical procedure.

Another advantage resides in visualizing planned medical procedure tasks and a probability for a remote expert or local technologist being interrupted by ad-hoc tasks as a function of time.

Another advantage resides in determining the availability of a remote technologist or radiologist based on their fixed tasks and their predicted ad-hoc tasks and by visualizing this information for a local technologist.

Another advantage resides in reduced errors, repeats, and reduced wasted time for medical procedures.

Another advantage resides in providing an electronic calendaring system that incorporates unscheduled interruptions in a probabilistic or statistical manner.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 diagrammatically shows an illustrative apparatus for managing a medical professional schedule in accordance with the present disclosure.

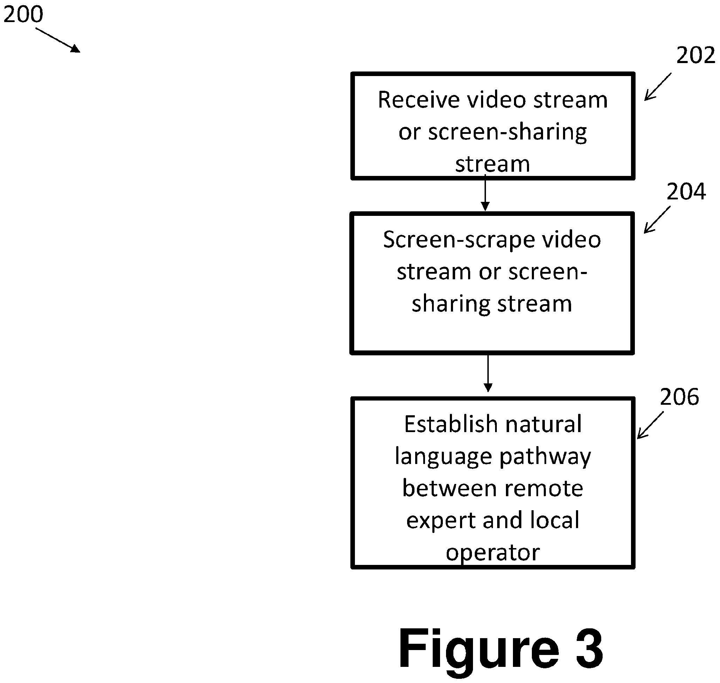

Figs. 2-4 show example flow charts of operations suitably performed by the apparatus of Fig. 1.
Figs. 5 and 6 show two examples of suitable output of the apparatus of Fig. 1.

### DETAILED DESCRIPTION OF EMBODIMENTS

This following relates to a system for predicting intervals during which a radiologist or remote technologist is likely (or unlikely) to be interrupted by ad hoc tasks, also referred to herein as interruptions. The disclosed system is configured to link radiologists in to provide rapid feedback on images or for other specialized tasks.

The disclosed system divides tasks into two categories: planned tasks and ad hoc tasks, also referred to as interruptions. Planned tasks are essentially anything that is known ahead of time so that it is (or at least could be) placed onto the calendar of the radiologist or remote technician. Ad hoc tasks arise unexpectedly and constitute interruptions in the planned schedule of the radiologist or remote technologist. Hence, the goal is to predict likelihood of interruption at any given time (or time block) in the course of a day. By definition, unscheduled interruptions cannot be incorporated into a calendaring system in a deterministic way. However, as disclosed herein, the likelihood of interruption for a given time or time block can be predicted in probabilistic fashion. This information can be conveyed to the user of the calendar in a suitable human-perceptible manner, such as by indicating time blocks of a rendering of the electronic calendar that have high probability of interruption using color coding highlighting or other visual indicator, or by marking such time blocks with an icon indicating a high probability of interruption, or so forth.

The interruption predictions are computed for each day, for each time block, or as a continuous a function of time in some embodiments, based on features of the day including for example the radiology lab schedule, profiles of patients, local technologists, remote technologists, and/or radiologists, and possibly other factors such as the time of day, day of week, day of year, and so forth. A machine learning (ML) tool is trained to predict the interruption likelihood given these inputs. In some embodiments, the ML tool computes this probability at any given time based on the inputs. As the inputs change over the course of the day as the radiology lab proceeds through its schedule, the interruption likelihood output by the ML tool will also vary over the day. The likelihood of interruption can be plotted as a continuous curve, or in another approach the likelihood of interruption could be computed for discrete time blocks, e.g., if imaging sessions are scheduled at 15-minute intervals then the likelihood of interruption could be computed for 15-minute time blocks.

In some embodiments, the inputs could include information from outside the time frame (e.g., instantaneous time point, or time block) for which the interruption likelihood is being computed. For example, if the probability is being computed for a radiology examination time slot N but the radiology examination in immediately preceding time slot N-1 is complex, then this could lead to a higher likelihood of interruption in time slot N since, for example, the technologist performing the complex radiology examination of time slot N-1 has a high likelihood of running into problems so that the N-1 time slot examination carries over into time slot N leading to a call to a remote tech during time slot N. This can be accounted for by including the exam in time slot N-1 as a feature input for computing likelihood of interruption for time slot N. In another approach, an explicitly time dependent ML model such as a time-dependent Markov model, a time-dependent finite element modeling (FEM), long-short-term memories (LSTM), other recurrent neural network (RNN) or so forth could be used for estimating the interruption probability as a function of time.

In some embodiments, the likelihood of interruption can factor into the personnel resourcing for a given schedule. It is often better practice and more cost-efficient to have sufficient staff with focus, as opposed to tasking the resource with too many functions and interruptions such that work cannot be reasonably and efficiently completed. Hence, time blocks with high likelihood of interruption may be beneficially staffed with additional personnel. In a variant embodiment, the likelihood of interruption can be recalculated for the higher number of personnel, and this process of computing likelihood of interruption and adjusting staff level as a function of time can be iterated to optimize the staff level as a function of time to optimize staffing to keep the likelihood of interruption as a function of time below some maximum acceptable interruption likelihood.

In other embodiments, the schedule of planned tasks can be used along with the computed probabilities of interruption to estimate a remote technologist availability score (or analogously a radiologist availability score). This information could be provided to the local technician to inform the latter of how quickly help can be received.

The output of the system could take various forms. In one approach, the remote technologist or radiologist could have an electronic calendar (e.g., a general-purpose commercial product such as an Outlook calendar provided by Microsoft, or an electronic calendar integrated into a Radiology Information System platform). The electronic calendar lists the planned tasks, with each time block of the calendar color-coded as to likelihood of interruption, or with the time blocks computed as having high likelihood of interruption being flagged with a suitable icon such as "(!)". Additionally or alternatively, the likelihood of interruption could be plotted as a function of time over the course of the day. Similarly, the local technologist could be presented with availability score plotted as a function of time over the course of the day.

The following illustrative examples are directed to a radiology domain, being described in terms of a remote expert assistance system for assigning an imaging technologist. Interruptions are particularly frequent in the radiology domain due to the close interactions between imaging technologists and radiologists, many of which are arise as ad hoc tasks that cannot be scheduled. For example, when an imaging technologist observes an unusual feature in an acquired image, the technologist may call a radiologist to receive instructions on how to proceed in view of this unusual feature. Such a call was not expected, and therefore cannot be scheduled on an electronic calendar. In radiology work settings with a dedicated remote expert assistance system, such interruptions may be even more frequent as unscheduled interruptions are facilitated by improved communication via the expert assistance system. While particularly useful in radiology domains, disclosed electronic calendaring systems and methods incorporating unscheduled interruptions as disclosed herein are expected to find more general application in electronic calendaring for medical professionals and for other workers or other persons susceptible to frequent interruptions.

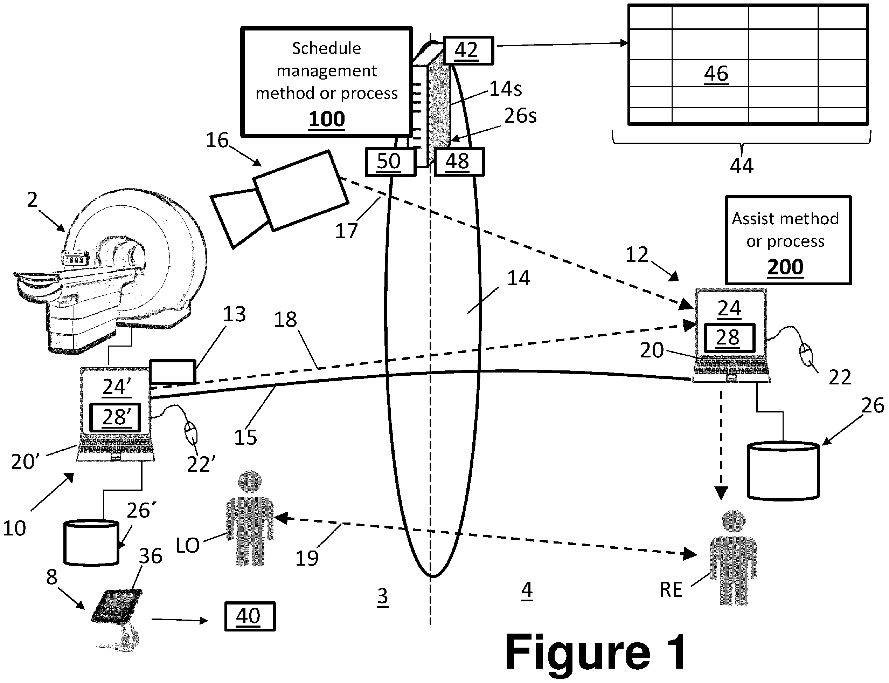

With reference to Fig. 1, an apparatus for providing assistance from a remote medical imaging expert RE to a local radiologist technician or local technician operator LO is shown. Such a system is also referred to herein as a radiology operations command center (ROCC). As shown in Fig. 1, the local operator LO, who operates a medical imaging device (also referred to as an image acquisition device, imaging device, and so forth) 2, is located in a medical imaging device bay 3, and the remote expert RE is disposed in a remote service location or center 4. It should be noted that the remote expert RE may not necessarily directly operate the medical imaging device 2, but rather provides assistance to the local operator LO in the form of advice, guidance, instructions, or the like.
The image acquisition device 2 can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device 2 may also be a hybrid imaging device such as a PET/CT or SPECT/CT imaging system. While a single image acquisition device 2 is shown by way of illustration in Fig. 1, more typically a medical imaging laboratory will have multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, the remote service center 4 may provide service to multiple hospitals. The local operator LO controls the medical imaging device 2 via an imaging device controller 10. The remote expert RE is stationed at a remote workstation 12 (or, more generally, an electronic controller 12).

The imaging device controller 10 includes an electronic processor 20', at least one user input device such as a mouse 22', a keyboard, and/or so forth, and a display device 24'. The imaging device controller 10 presents a device controller graphical user interface (GUI) 28' on the display 24' of the imaging device controller 10, via which the local operator LO accesses device controller GUI screens for entering the imaging examination information such as the name of the local operator LO, the name of the patient and other relevant patient information (e.g. gender, age, etc.) and for controlling the (typically robotic) patient support to load the patient into the bore or imaging examination region of the imaging device 2, selecting and configuring the imaging sequence(s) to be performed, acquiring preview scans to verify positioning of the patient, executing the selected and configured imaging sequences to acquire clinical images, display the acquired clinical images for review, and ultimately store the final clinical images to a Picture Archiving and Communication System (PACS) or other imaging examinations database. In addition, while Fig. 1 shows a single medical imaging device bay 3, it will be appreciated that the remote service center 4 (and more particularly the remote workstation 12) is in communication with multiple medical bays via a communication link 14, which typically comprises the Internet augmented by local area networks at the remote operator RE and local operator LO ends for electronic data communications. Consequently, the remote operator RE is susceptible to unplanned interruptions as the imaging technologists working in these numerous imaging bays may experience unexpected difficulties and call for assistance from the remote operator RE.

As diagrammatically shown in Fig. 1, in some embodiments, a camera 16 (e.g., a video camera) is arranged to acquire a video stream 17 of a portion of the medical imaging device bay 3 that includes at least the area of the imaging device 2 where the local operator LO interacts with the patient, and optionally may further include the imaging device controller 10. The video stream 17 is sent to the remote workstation 12 via the communication link 14, e.g., as a streaming video feed received via a secure Internet link.

In other embodiments, the live video feed 17 of the display 24' of the imaging device controller 10 is, in the illustrative embodiment, provided by a video cable splitter 15 (e.g., a DVI splitter, a HDMI splitter, and so forth). In other embodiments, the live video feed 17 may be provided by a video cable connecting an auxiliary video output (e.g. aux vid out) port of the imaging device controller 10 to the remote workstation 12 of the operated by the remote expert RE. Alternatively, a screen mirroring data stream 18 is generated by screen sharing software 13 running on the imaging device controller 10 which captures a real-time copy of the display 24' of the imaging device controller 10, and this copy is sent from the imaging device controller 10 to the remote workstation 12. These are merely nonlimiting illustrative examples.

The communication link 14 also provides a natural language communication pathway 19 for verbal and/or textual communication between the local operator LO and the remote expert RE, in order to enable the latter to assist the former in performing the imaging examination. For example, the natural language communication link 19 may be a Voice-Over-Internet-Protocol (VOIP) telephonic connection, a videoconferencing service, an online video chat link, a computerized instant messaging service, or so forth. Alternatively, the natural language communication pathway 19 may be provided by a dedicated communication link that is separate from the communication link 14 providing the data communications 17, 18, e.g., the natural language communication pathway 19 may be provided via a landline telephone. These are again merely nonlimiting illustrative examples.

Fig. 1 also shows, in the remote service center 4 including the remote workstation 12, such as an electronic processing device, a workstation computer, or more generally a computer, which is operatively connected to receive and present the video 17 of the medical imaging device bay 3 from the camera 16 and to present the screen mirroring data stream 18 as a mirrored screen. Additionally or alternatively, the remote workstation 12 can be embodied as a server computer or a plurality of server computers, e.g., interconnected to form a server cluster, cloud computing resource, or so forth. The workstation 12 includes typical components, such as an electronic processor 20 (e.g., a microprocessor), at least one user input device (e.g., a mouse, a keyboard, a trackball, and/or the like) 22, and at least one display device 24 (e.g., an LCD display, plasma display, cathode ray tube display, and/or so forth). In some embodiments, the display device 24 can be a separate component from the workstation 12. The electronic processor 20 is operatively connected with a one or more non-transitory storage media 26. The non-transitory storage media 26 may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation 12, various combinations thereof, or so forth. It is to be understood that any reference to a non-transitory medium or media 26 herein is to be broadly construed as encompassing a single medium or multiple media of the same or different types. Likewise, the electronic processor 20 may be embodied as a single electronic processor or as two or more electronic processors. The non-transitory storage media 26 stores instructions executable by the at least one electronic processor 20. The instructions include instructions to generate a graphical user interface (GUI) 28 for display on the remote operator display device 24.

The medical imaging device controller 10 in the medical imaging device bay 3 also includes similar components as the remote workstation 12 disposed in the remote service center 4. Except as otherwise indicated herein, features of the medical imaging device controller 10 disposed in the medical imaging device bay 3 similar to those of the remote workstation 12 disposed in the remote service center 4 have a common reference number followed by a "prime" symbol (e.g., processor 20', display 24', GUI 28') as already described. In particular, the medical imaging device controller 10 is configured to display the imaging device controller GUI 28' on a display device or controller display 24' that presents information pertaining to the control of the medical imaging device 2 as already described, such as imaging acquisition monitoring information, presentation of acquired medical images, and so forth. The real-time copy of the display 24' of the controller 10 provided by the video cable splitter 15 or the screen mirroring data stream 18 carries the content presented on the display device 24' of the medical imaging device controller 10. The communication link 14 allows for screen sharing from the display device 24' in the medical imaging device bay 3 to the display device 24 in the remote service center 4. The GUI 28' includes one or more dialog screens, including, for example, an examination/scan selection dialog screen, a scan settings dialog screen, an acquisition monitoring dialog screen, among others. The GUI 28' can be included in the video feed 17 or provided by the video cable splitter 15 or by the mirroring data stream 17' and displayed on the remote workstation display 24 at the remote location 4.

Fig. 1 shows an illustrative local operator LO, and an illustrative remote expert RE. However, the ROCC optionally provides a staff of remote experts who are available to assist local operators LO at different hospitals, radiology labs, or the like. The ROCC may be housed in a single physical location or may be geographically distributed. The server computer 14s is operatively connected with a one or more non-transitory storage media 26s. The non-transitory storage media 26s may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer 14s, various combinations thereof, or so forth. It is to be understood that any reference to a non-transitory medium or media 26s herein is to be broadly construed as encompassing a single medium or multiple media of the same or different types. Likewise, the server computer 14s may be embodied as a single electronic processor or as two or more electronic processors. The non-transitory storage media 26s stores instructions executable by the server computer 14s. In addition, the non-transitory computer readable medium 26s (or another database) stores data related to a set of remote experts RE and/or a set of local operators LO. The remote expert data can include, for example, skill set data, work experience data, data related to ability to work on multi-vendor modalities, data related to experience with the local operator LO and so forth.

In addition, the server 14s is configured to store a schedule 42 (sometimes also referred to as a calendar 42) of one or more medical professionals. The schedule or calendar 42 includes time intervals 44 with planned tasks for the remote expert RE and/or the local operator LO. The planned tasks can include, for example, one or more of a lab schedule, patient profiles, local technologists' profiles, remote technologists' profiles, time of day of the task, and day of year of the task. It will be appreciated that an "interruption" in this context is thus an unscheduled or unplanned task that is not on the schedule 42 of planned tasks.

Furthermore, as disclosed herein the server 14s implements an electronic calendaring system that performs a method or process 100 of managing the schedule 42 of a medical professional (i.e., an electronic calendar method 100). In illustrative examples, the schedule 42 is assumed to be the calendar of the remote expert RE, as a nonlimiting example. The electronic calendaring system performing the method or process 100 can be implemented as a commercial calendaring system (e.g., Microsoft Outlook, Google Calendar, et cetera) that is modified as disclosed herein to incorporate unplanned interruptions. In other embodiments, the electronic calendaring system performing the method or process 100 may be integrated into the ROCC system, which advantageously facilitates automatically populating the schedule or calendar 42 with ROCC-related tasks such as imaging examinations assigned to the remote expert RE.

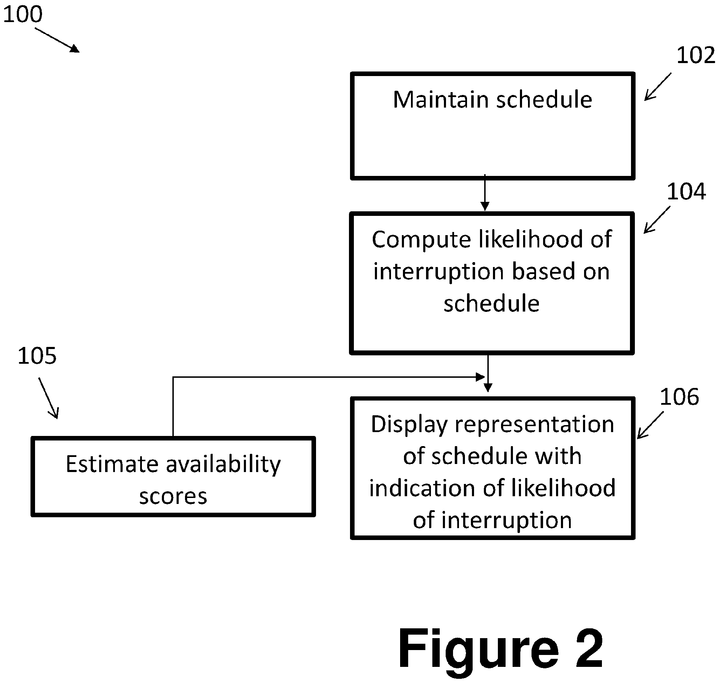

With reference to Fig. 2, and with continuing reference to Fig. 1, an illustrative embodiment of the method 100 is diagrammatically shown as a flowchart. At an operation 102, an electronic calendar (i.e., the schedule or calendar 42) that includes assigned tasks for an owner (e.g., the remote expert RE or the local operator LO) of the electric calendar to time intervals of the electronic calendar is maintained in the server 14s. This maintenance includes adding, updating or removing (as may be appropriate), tasks. The addition, modification, or removal of tasks can be done manually, for example using a user interface (UI) via which the owner can add, modify, or remove tasks. In a typical UI workflow, the owner clicks on a time slot to create a task, which brings up a dialog window into which the owner can enter or modify the time interval, add a task title and/or description, and optionally add other relevant information such as creating a videoconference link for the task, sharing the task with other users (e.g., of the owner is creating a meeting task, it may be shared with other meeting participants), or so forth. Additionally or alternatively, calendar tasks may be created automatically, for example by way of receiving a meeting invite from another user (which may need to be accepted by the owner of the schedule 42 before it is added to the schedule 42), or tasks may be automatically added by the ROCC system in the illustrative ROCC context. In some embodiments, tasks may be added either manually or automatically. Moreover, it is to be appreciated that the operation 102 is typically ongoing, in that the tasks may be added, modified, or deleted at any time.

At an operation 104, a likelihood of interruption is computed as a function of time. For example, the likelihood of interruption can be computed for time blocks 46 of the calendar 42 based on historical information on interruptions of the owner of the electronic calendar 42. In another example, the likelihood of interruption can be computed for time blocks 46 of the calendar 42 based on schedules of tasks for others that interact with the owner of the electronic calendar. The schedules of tasks for others that interact with the owner may be obtained from calendars maintained by the schedule management method or process 100 for those others that interact with the owner. Additionally or alternatively, the schedules of tasks for others that interact with the owner may be received from some other calendar system. In some embodiments, the operation 104 computes the likelihood of interruption as a function of time over a course of a day in the schedule 42.

In some embodiments, the computing operation 104 can include applying a machine learning (ML) component 48 to the maintained or received other schedules to compute the likelihood of interruption as a function of time. For example, the ML component 48 can comprise a time-dependent Markov model or a time-dependent finite element model, a long-short-term memories (LSTM), a recurrent neural network (RNN), and so forth. In such embodiments, the historical information on interruptions of the owner of the electronic calendar 42 may be used to train the ML component 48.

In some embodiments, the computing operation 104 can include computing the potential interruption predictions with information outside of a time block 46 of the schedule 42 for which the potential interruption prediction for each time block 46. To do so, the potential interruption prediction is computed for a first time block 46 and for a second time block 46 adjacent the first time block. A determination is then made whether the computed potential interruption prediction for the first time block 46 should be used for computing the potential interruption prediction for the second time block 46.

At an (optional) operation 105, an availability score 50 of a remote medical professional (i.e., the remote expert RE) is estimated based on the schedule 42 and the computed potential interruption prediction for each time block 46.

At an operation 106, a representation 40 of the schedule 42 of the medical professional with an indication of the likelihood of interruption as a function of time in the schedule 42 is output on the display device 36 of the ROCC device 8 (for visualization by the local operator LO) or on the display device 24 of the remote workstation 12 (for visualization by the remote expert RE). For example, the representation 40 of the schedule 42 can be annotated to the corresponding time intervals 44 and further including indications of the computed likelihoods of interruption annotated to the corresponding time blocks 46. To do so, the time blocks 46 are annotated with their respective likelihood of interruption values by color-coding the time blocks 46 in accordance with their respective likelihood of interruption values. In one example, the likelihood of interruption can be plotted as a function of time as a continuous curve (i.e., over the day of the schedule 42). In another example, the likelihood of interruption can be plotted as a discrete time block representation for the time intervals 46 of the schedule 42. In another example, the representation 40 can include the availability score 50 plotted as a function of time for the day of the schedule 42.

In one example embodiment, when the schedule 42 comprises the calendar with time blocks 46, the likelihood of interruption as a function of time is computed as likelihood of interruption values for the respective time blocks 46 of the calendar 42, and the representation 40 of the schedule 42 comprises a representation of the calendar 42 with the time intervals with planned tasks annotated and the time blocks 46 annotated with their respective likelihood of interruption values.

Referring back to Fig. 1, when the local operator LO requires assistance from the remote expert RE, the communication link 14 connects the local operator LO/ remote expert RE. The GUI 28 is provided as a remote assistance UI on the display device 24 operable by a remote expert RE. The UI 28 provides two-way communication between the local operator LO and the remote expert RE via which the remote expert can provide assistance to the local medical imaging device operator LO.

The remote workstation 12 of the selected remote expert RE, and/or the medical imaging device controller 10 being run by the local operator LO, is configured to perform a method or process 200 for providing assistance from the remote expert RE to the local operator LO. For brevity, the method 200 will be described as being performed by the remote workstation 12. The non-transitory storage medium 26 stores instructions which are readable and executable by the at least one electronic processor 20 (of the workstation 12, as shown, and/or the electronic processor or processors of a server or servers on a local area network or the Internet) to perform disclosed operations including performing the method or process 200.

A suitable implementation of the assistance method or process 200 is as follows. The method 200 is performed over the course of (at least a portion of) an imaging procedure performed using the medical imaging device 2, and the remote expert RE is one selected via the method 100 having availability. For example, a remote expert RE having the most availability (i.e., being the least likely to be interrupted), having a required expertise (i.e., to ensure quality resources for assistance, and avoid repeated interruptions). In another example, the interruptions can be load balanced over the available remote experts RE. To perform the method 200, at an operation 202, the workstation 12 in the remote location 4 is programmed to receive at least one of: (i) the video 17 from the video camera 16 of the medical imaging device 2 located in the medical imaging device bay 3; and/or (ii) the screen sharing 18 from the screen sharing software 13; and/or (iii) the video 17 tapped by the video cable splitter 15. The video feed 17 and/or the screen sharing 18 can be displayed at the remote workstation display 24, typically in separate windows of the GUI 28. At an operation 204, the video feed 17 and/or the screen sharing 18 can be screen-scraped to determine information related to the medical imaging examination (e.g., modality, vendor, anatomy to be imaged, cause of issue to be resolved, and so forth). In particular, the GUI 28 presented on the display 24 of the remote workstation 12 preferably includes a window presenting the video 17, and a window presenting the mirrored screen of the medical imaging device controller 10 constructed from the screen mirroring data stream 18, and status information on the medical imaging examination that is maintained at least in part using the screen-scraped information. This allows the remote operator RE to be aware of the content of the display of the medical imaging device controller 10 (via the shared screen) and also to be aware of the physical situation, e.g., position of the patient in the medical imaging device 2 (via the video 17), and to additionally be aware of the status of the imaging examination as summarized by the status information. During an imaging procedure, at an operation 206, the natural language communication pathway 19 is established between the remote medical professional workstation 12 and the ROCC device 8, and is suitably used to allow the local operator LO and the remote operator RE to discuss the procedure and in particular to allow the remote operator to provide advice to the local operator LO.

In some examples, data can be collected based on the issue with the medical device 2, a state of the medical device 2, or an imaging protocol. This data can be prepared in advance of contacting a remote expert RE to assist the local operator LO.

### EXAMPLE

The following describes another example of the method 300. Planned tasks are known to happen at a certain time based on a previously defined or predicted schedule of activities. For example, a planned task of a radiologist may be a legally required image quality check for a specific examination or a scheduled meeting or conference. An ad-hoc task is a task that may or may not happen and typically is requested on short notice. For example, if a local operator LO runs into unexpected problems with a geometry definition, they may need to call a radiologist or remote expert RE for help.

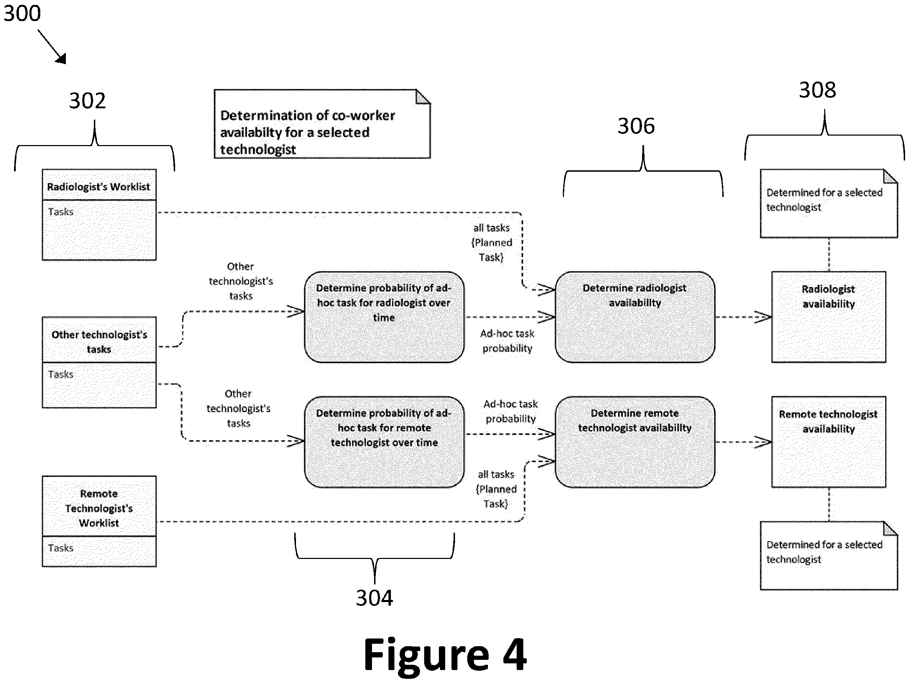

Fig. 4 shows another example of the method 300. At an operation 302, worklists of a radiologist, a remote technologist, and other local technologists are maintained. At an operation 304, probabilities of ad-hoc tasks for the radiologist and the remote technologist (i.e., interruptions) are computed overtime. Thus, operation 304 corresponds to operation 104 of the embodiment of Fig. 2. At an operation 306, availabilities are determined for the radiologist and the remote technologist. At an operation 308, the availabilities are output.

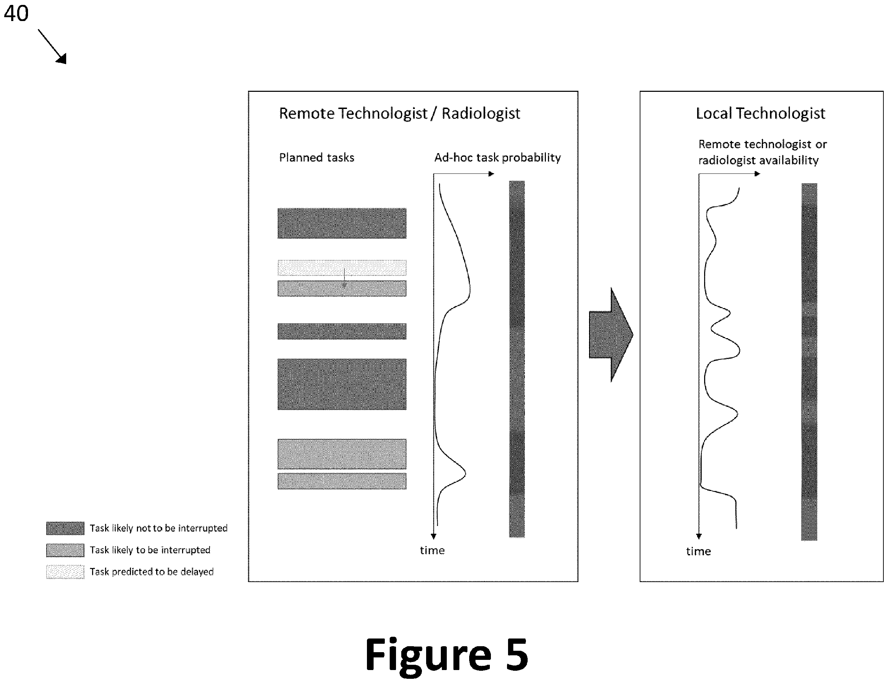

Fig. 5 shows an example of the visualization 40 of planned tasks in a calendar-like view with fixed items. Even known delays in the schedule 42 can be included in the visualization 40 by moving the respective items to a different time. The occurrence of ad-hoc tasks cannot be predicted with certainty, but based on historic data, a probability for ad-hoc task occurrence can be specified. This is ad-hoc task probability is visualized as shown in Fig. 5 as, for example, a curve or a color-coded bar.

The visualization 40 allows allow the radiologist or remote technologist to know in advance when to expect to be disturbed frequently and when to expect time periods with fewer ad-hoc requests. In this way, the radiologist or technologist is enabled to adapt the planning of their day accordingly, e.g., by starting complex reading tasks only during low ad-hoc task probability periods, or by moving their lunch break to a time when they are most likely not required to perform an ad-hoc task.

Having information about both planned tasks and ad-hoc task probability, the overall occupation of the radiologist or remote technologist can be calculated, for example by assigning an occupation probability of 1 to the scheduled task periods and calculating a weighted sum of planned task occupation probability and ad-hoc task probability. The weighting factors can be adapted to reflect the effort required for planned and ad-hoc tasks. If different planned tasks require different effort or concentration level of the radiologist or remote technologist, weighting factors can be chosen differently for different types of tasks.

The overall occupation can be translated to an availability level of the radiologist or remote technologist. The availability level may be expressed as one minus the occupation probability. The availability level can be visualized for the local technologist as a function of time as shown in Fig. 5. Again, a representation as a curve or a color-coded bar are possible implementations. The visualization 40 allows the local technologist to decide when the best time for communicating with a radiologist or remote technologist would be. Requests or communication that are not time-critical can then be postponed to a time where the chance for causing disruptions and disturbances is lowest.

The ad-hoc task probability (i.e., probability of interruption) for the remote technologist or radiologist refers to imaging examination tasks on the local technologist's worklist and may be determined by, for example, a type of procedures and protocols scheduled at imaging modalities, patient characteristics (age, weight, special needs, etc.), patient's medical history (for determination of probability for incidental findings), an experience level of local technologist operating the image modality, an availability of other staff in the imaging department (e.g. more experienced staff, staff shortage due to illness, etc.), standard operating procedures and legal requirements, and so forth. For example, a less-experienced technologist may have a high probability for requesting help from a remote technologist 12 minutes into a cardiac examination of a paediatric patient, when a difficult geometry planning task needs to be performed.

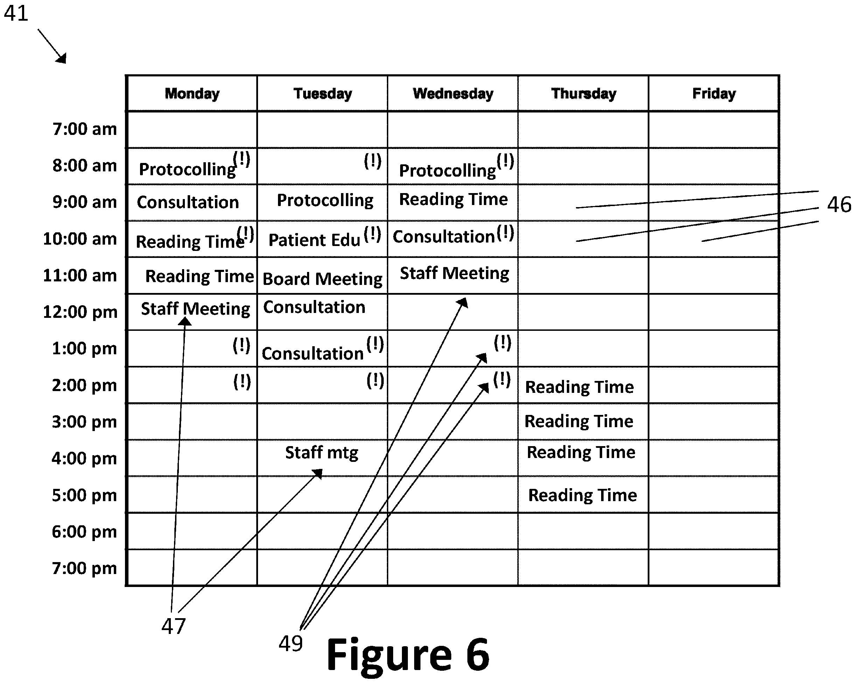

With reference to Fig. 6, another nonlimiting illustrative visualization 40 comprises a calendar comprising time blocks 46. The likelihood of interruption as a function of time are computed as likelihood of interruption values for the respective time blocks 46 of the calendar, and the embodiment of the representation 40 of the schedule of the medical professional shown in Fig. 6 comprises the representation of the calendar with the time intervals with planned tasks annotated (e.g., as annotations 47 for "Protocolling," "Reading Time", "Consultation," "Board Meeting," "Patient Education," and "Staff Meeting") and the time blocks annotated with their respective likelihood of interruption values by color coding or, in the illustrative example of Fig. 6, by icons 49 labeling time blocks 46 with high likelihood of interruption.

Probabilities for interruptions comprising ad-hoc requests can be estimated manually for each combination of protocol, sequence, patient type, and operator experience. A preferred solution, however, would be to employ the ML component 48 to estimate the ad-hoc task probability based on historic data. The ML component 48 can comprise, for example, a neural network, a decision tree, a statistical model, a random forest algorithm, a support vector machine, or similar algorithm.

For training the ML component 48, a feature set consisting of the above-mentioned information determining the ad-hoc task probability needs to be supplied ("features"), as well as information about the actually requested ad-hoc tasks and the points in time when the tasks were requested ("labels"). For each imaging examination, the ML component 48 can then be able to predict the probability for an ad-hoc request for each point in time during/before/after the examination. In one embodiment, the imaging examination is further broken down into multiple activities, such as patient positioning, geometry planning, and (in the case of MR) individual sequences. The determination of the ad-hoc task probability can then be performed for each individual activity.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

In the foregoing detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

## Claims

1. A non-transitory computer readable medium (26s) storing instructions readable and executable by at least one electronic processor (14s) to perform a method (100) managing a schedule (42) of a medical professional, the method comprising:
maintaining a schedule of tasks for the medical professional, the schedule including time intervals (44) with planned tasks;
maintaining or receiving other schedules of tasks for others that interact with the medical professional;
computing a likelihood of interruption of the medical professional as a function of time from the maintained or received other schedules; and
outputting, on a display device (24, 36) of an electronic processing device (12, 8) operable by the medical professional, a representation (40) of the schedule of the medical professional with an indication of the likelihood of interruption as a function of time in the schedule.

2. The non-transitory computer readable medium (26s) of claim 1, wherein the computing of the likelihood of interruption as a function of time includes:
applying a machine learning (ML) component (48) to the other schedules to compute the likelihood of interruption as a function of time.

3. The non-transitory computer readable medium (26s) of claim 2, wherein the likelihood of interruption as a function of time is computed over a course of a day in the schedule (42).

4. The non-transitory computer readable medium (26s) of any one of claims 1-3, wherein the method (100) further includes:
plotting the likelihood of interruption as a function of time as a continuous curve; and
outputting the continuous curve on the display device (24, 36).

5. The non-transitory computer readable medium (26s) of any one of claims 1-3, wherein the method (100) further includes:
plotting the likelihood of interruption as a function of time as a discrete time block representation for the time intervals (44) of the schedule (42);
outputting the discrete time block representation on the display device (24, 36).

6. The non-transitory computer readable medium (26s) of any one of claims 1-5, wherein computing potential interruption predictions from the maintained or received other schedules includes:
computing the potential interruption predictions with information from the maintained or received other schedules outside of a time block (46) for which the potential interruption prediction is computed.

7. The non-transitory computer readable medium (26s) of claim 6, wherein computing the potential interruption predictions with information from the maintained or received other schedules outside of a time block (46) for which the potential interruption prediction is computed includes:
computing the potential interruption prediction for a first time block;
computing the potential interruption prediction for a second time block adjacent the first time block;
determining whether the computed potential interruption prediction for the first time block should be used for computing the potential interruption prediction for the second time block.

8. The non-transitory computer readable medium (26s) of any one of claims 2-7, wherein the ML component (48) comprises one of a time-dependent Markov model, a time-dependent finite element model, a time-dependent finite element modeling (FEM), long-short-term memories (LSTM), or a recurrent neural network (RNN).

9. The non-transitory computer readable medium (26s) of any one of claims 1-8, wherein the method (100) further includes:
estimating an availability score (50) of a medical professional based on the schedule (42) and the computed potential interruption prediction for each time block (46).

10. The non-transitory computer readable medium (26s) of any one of claims 1-9, wherein:
the schedule (42) comprises a calendar comprising time blocks (46);
the likelihood of interruption as a function of time is computed as likelihood of interruption values for the respective time blocks of the calendar; and
the representation (40) of the schedule of the medical professional comprises a representation of the calendar with the time intervals with planned tasks annotated and the time blocks annotated with their respective likelihood of interruption values.

11. The non-transitory computer readable medium (26s) of claim 10, wherein the time blocks (46) are annotated with their respective likelihood of interruption values by color-coding the time blocks in accordance with their respective likelihood of interruption values.

12. The non-transitory computer readable medium (26s) of any one of claims 1-9, wherein outputting, on a display device (24, 36) of an electronic processing device (12, 8) operable by a medical professional, a representation (40) of the schedule (42) of the medical professionals with an indication of a likelihood of interruption for the time blocks (46) in the schedule includes one of:
outputting the representation as a time curve over the day of the schedule; or
outputting the representation as the availability score (50) plotted as a function of time for the day of the schedule.

13. The non-transitory computer readable medium (26s) of any one of claims 1-12, further storing instructions executable by at least one electronic processor (14s) to perform a remote assistance method (200) including:
providing two-way communication between a remote medical professional workstation (12) and an electronic device (8) disposed with a medical device during a planned task in the schedule (42).

14. The non-transitory computer readable medium (26s) of any one of claims 1-13, wherein the likelihood of interruption comprises a likelihood of an unplanned task that is not on the schedule (42) of tasks.

15. An electronic calendar method (100) comprising:
maintaining an electronic calendar (42) including assigning tasks for an owner of the electric calendar to time intervals of the electronic calendar;
estimating likelihoods of interruption of the owner of the electronic calendar for time blocks (46) of the calendar based on schedules of tasks for others that interact with the owner of the electronic calendar; and
displaying, on a display device (24, 36) controlled by the at least one electronic processor, a representation (40) of the electronic calendar including the tasks assigned to the owner of the electronic calendar annotated to the corresponding time intervals and further including indications of the computed likelihoods of interruption annotated to the corresponding time blocks.
